# EUROPEAN PATENT APPLICATION

(11) **EP 2 105 094 A2**
(43) Date of publication of application: **30.09.2009**
(21) Application number: 09004498.3
(22) Date of filing: 27.03.2009
(51) Int. Cl.: A61B 8/13, A61B 8/12

(54) **Adapter and ultrasonic diagnosis system**

(30) Priority: 28.03.2008 JP 2008088713
(71) Applicant: Fujifilm Corporation, Tokyo 106-0031 (JP)
(72) Inventor: Tanaka, Toshizumi, Saitama-shi Saitama (JP)
(74) Representative: Höhfeld, Jochen

(57) **Abstract**

An ultrasonic diagnosis system includes an ultrasonic diagnosis device (30) for diagnosis according to a signal from a first ultrasonic probe (60) for extracorporeal use. A second ultrasonic probe (10, 82) detects an object for in-vivo diagnosis. An adapter inputs and/or outputs between the ultrasonic diagnosis device and the second ultrasonic probe. The adapter includes a holder chamber (71, 186) for holding the ultrasonic diagnosis device. A diagnosis device connector (74) is for connection with the ultrasonic diagnosis device. A probe connector (72) is for connection with the second ultrasonic probe. An input/output device (120) is for transmission and/or reception between the probe connector and the diagnosis device connector. In addition to the ultrasonic diagnosis device, a picture-in-picture processing unit operates for picture-in-picture processing of an ultrasonic or endoscopic image. Furthermore, a processor connector is for connection with an endoscope processor for receiving a signal from the endoscope to create an endoscopic image.

## Description

### BACKGROUND OF THE INVENTION

### 1. Field of the Invention

The present invention relates to an adapter and an ultrasonic diagnosis system. More particularly, the present invention relates to an adapter and an ultrasonic diagnosis system in which extracorporeal device diagnosis and in-vivo diagnosis of a human cavity of a patient's body are possible in a single system in a simplified manner.

### 2. Description Related to the Prior Art

Ultrasonic diagnosis in the medical field is a non-invasive diagnosis for applying ultrasonic waves to an object in a patient's body, to observe a state of the object by receiving and imaging echo ultrasonically reflected from the object. There are two types of ultrasonic diagnosis including extracorporeal device diagnosis for applying the ultrasonic waves through the skin of the body, and in-vivo diagnosis for applying the ultrasonic waves upon entry of a probe in a human cavity. It is possible in the in-vivo diagnosis to examine the tissue of the human cavity or gastrointestinal tract more precisely than in the extracorporeal device diagnosis. The in-vivo diagnosis is important specifically for diagnosing the depth of ulcer, tumor and the like in the body.

An ultrasonic diagnosis system for the extracorporeal device diagnosis includes an ultrasonic probe, an ultrasonic diagnosis device, and a display panel. The ultrasonic probe has a scan head containing ultrasonic transducers for emitting the ultrasonic waves and receiving echo ultrasonically reflected from an object. The ultrasonic diagnosis device retrieves a detection signal from the echo from the object by control of the ultrasonic probe, and creates an ultrasonic image according to the detection signal. The display panel displays the ultrasonic image created by the ultrasonic diagnosis device. U.S.P. Nos. 6,364,839 and 6,447,451 (corresponding to JP-A 2002-542870) disclose the ultrasonic diagnosis system including a portable type of the ultrasonic diagnosis device in which the display panel is assembled on a device body. It is possible to examine a patient even on the bed side with the ultrasonic diagnosis device, which can be a general-purpose device for use in the extracorporeal device diagnosis.

The ultrasonic diagnosis system for the in-vivo diagnosis by use of an ultrasonic endoscope includes ultrasonic transducers and a CCD in its head assembly. JP-A 10-211201 discloses the ultrasonic diagnosis system which includes the ultrasonic endoscope, an endoscope processor, an ultrasonic processor for in-vivo diagnosis, and the display panel. The endoscope processor operates for control of the CCD, and creates an endoscopic image. The ultrasonic processor operates for control of the ultrasonic transducers and creates the ultrasonic image.

An ultrasonic diagnosis device of extracorporeal use and the ultrasonic processor for creating the ultrasonic image are so expensive that a medical cost of a hospital will be considerably high for the purpose of both of the extracorporeal device diagnosis and the in-vivo diagnosis in the same facilities. It is conceivable to carry out the extracorporeal device diagnosis and the in-vivo diagnosis in one system or apparatus by control of both of the ultrasonic probe and the ultrasonic endoscope.

Differences in the performance and the apparatus size between the ultrasonic diagnosis device of extracorporeal use and the ultrasonic processor are a serious problem in considering a single system for both types of the examinations. It is necessary for the ultrasonic processor to have a function for creating the ultrasonic image by control of the ultrasonic endoscope and also a function for interfacing with the endoscope processor. In contrast, the ultrasonic diagnosis device of extracorporeal use can only have a function for creating the ultrasonic image by control of the ultrasonic probe. The ultrasonic processor requires a greater number of functions than the ultrasonic diagnosis device of extracorporeal use. It is very difficult to construct the ultrasonic processor compactly in an easily portable manner unlike the structure of the ultrasonic diagnosis device of extracorporeal use.

If the ultrasonic diagnosis system is constructed in compliance with performance of the ultrasonic processor, the size of the ultrasonic diagnosis system as a composite apparatus will be so large that placement of the same to a different position is very difficult. The general-purpose property of the ultrasonic diagnosis system for the extracorporeal device diagnosis will be low. If the ultrasonic diagnosis system is constructed in compliance with performance of the ultrasonic diagnosis device of extracorporeal use, the in-vivo diagnosis will be very complicated because of lack of interfacing with the endoscope processor, despite its general-purpose property in view of the extracorporeal device diagnosis.

### SUMMARY OF THE INVENTION

In view of the foregoing problems, an object of the present invention is to provide an adapter and an ultrasonic diagnosis system in which extracorporeal device diagnosis and in-vivo diagnosis of a human cavity of a patient's body are possible in a single system in a simplified manner.

In order to achieve the above and other objects and advantages of this invention, an adapter includes a holder chamber for holding an ultrasonic diagnosis device adapted to diagnosis according to a signal from a first ultrasonic probe for extracorporeal use. There is a diagnosis device connector for connection with the ultrasonic diagnosis device. A probe connector is for connection with a second ultrasonic probe for in-vivo diagnosis to detect an object. An input/output device inputs and/or outputs between the ultrasonic diagnosis device and the second ultrasonic probe through the probe connector and the diagnosis device connector. There is an auxiliary device for performance auxiliary to the ultrasonic diagnosis device.

The diagnosis device connector is connectable with a connection terminal of the ultrasonic diagnosis device for the first ultrasonic probe.

The holder chamber is shaped in a box-shaped recessed form, and the diagnosis device connector is positioned on an inner surface of the holder chamber.

The ultrasonic diagnosis device includes a lower surface, a first lateral surface oriented erectly from the lower surface, and a second lateral surface oriented transversely with the first lateral surface. The holder chamber includes a support surface for supporting the lower surface, a first inner surface oriented erectly from the support surface and opposed to the first lateral surface, and a second inner surface oriented erectly from the support surface and opposed to the second lateral surface. The connection terminal is disposed on the first lateral surface, and the diagnosis device connector is disposed on the first inner surface.

Furthermore, there is a processor connector for connection with an endoscope processor for receiving a signal from an endoscope to create an endoscopic image.

The second ultrasonic probe is disposed on the endoscope. The endoscope includes an image sensor for picking up the object and outputting a signal to the endoscope processor.

The auxiliary device includes a display control unit for control of displaying at least one of an ultrasonic image from the ultrasonic diagnosis device and the endoscopic image from the endoscope processor.

The auxiliary device includes a picture-in-picture processing unit for picture-in-picture processing of an ultrasonic image or the endoscopic image.

The auxiliary device includes a superimposer for control of superimposing alphanumerical information or a patterned line on an ultrasonic image or the endoscopic image.

Furthermore, there is an input interface for inputting a command signal for operation.

Furthermore, a battery charger charges a battery contained in the ultrasonic diagnosis device.

Furthermore, a casing contains the input/output device and the auxiliary device. The holder chamber is formed with the casing.

In one embodiment, furthermore, a casing contains the input/output device and the auxiliary device. The holder chamber is separate from the casing.

The second ultrasonic probe includes an ultrasonic transducer.

A number of signal lines between the ultrasonic diagnosis device and the diagnosis device connector is smaller than a number of signal lines between the second ultrasonic probe and the probe connector.

The input/output device includes a multiplexer and lines for interconnecting the multiplexer and the signal lines, and the multiplexer changes over line connection between the signal lines.

In one aspect of the invention, an ultrasonic diagnosis system is provided, and includes an ultrasonic diagnosis device for diagnosis according to a signal from a first ultrasonic probe for extracorporeal use. There is a second ultrasonic probe for in-vivo diagnosis to detect an object. An adapter inputs and/or outputs between the ultrasonic diagnosis device and the second ultrasonic probe. The adapter includes a holder chamber for holding the ultrasonic diagnosis device. A diagnosis device connector is for connection with the ultrasonic diagnosis device. A probe connector is for connection with the second ultrasonic probe. An input/output device is for transmission and/or reception between the probe connector and the diagnosis device connector. An auxiliary device is for performance auxiliary to the ultrasonic diagnosis device.

Accordingly, extracorporeal device diagnosis and in-vivo diagnosis of a human cavity of a patient's body are possible in a single system in a simplified manner, because the input/output device operates to couple the second ultrasonic probe with the ultrasonic diagnosis device and can have a shape easy to use in the ultrasonic diagnosis system.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an ultrasonic diagnosis system;
Fig. 2 is a perspective view illustrating a portable ultrasonic diagnosis device;
Fig. 3 is a perspective view illustrating an adapter;
Fig. 4 is a block diagram schematically illustrating an inner structure of the ultrasonic diagnosis system;
Fig. 5A is a front elevation illustrating an example of a first combined image;
Fig. 5B is a front elevation illustrating a second combined image;
Fig. 6 is a block diagram schematically illustrating connection between pulsers, receivers and ultrasonic transducers;
Fig. 7 is a perspective view illustrating another preferred adapter in which a holder chamber is separate from the body.

### DETAILED DESCRIPTION OF THE PREFERRED EMBODIMENT(S) OF THE PRESENT INVENTION

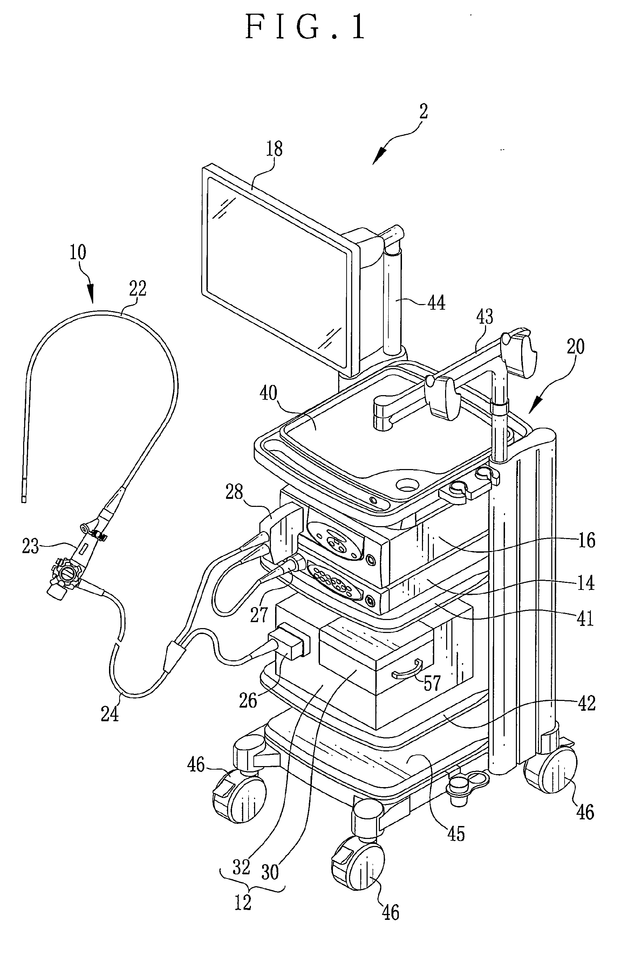

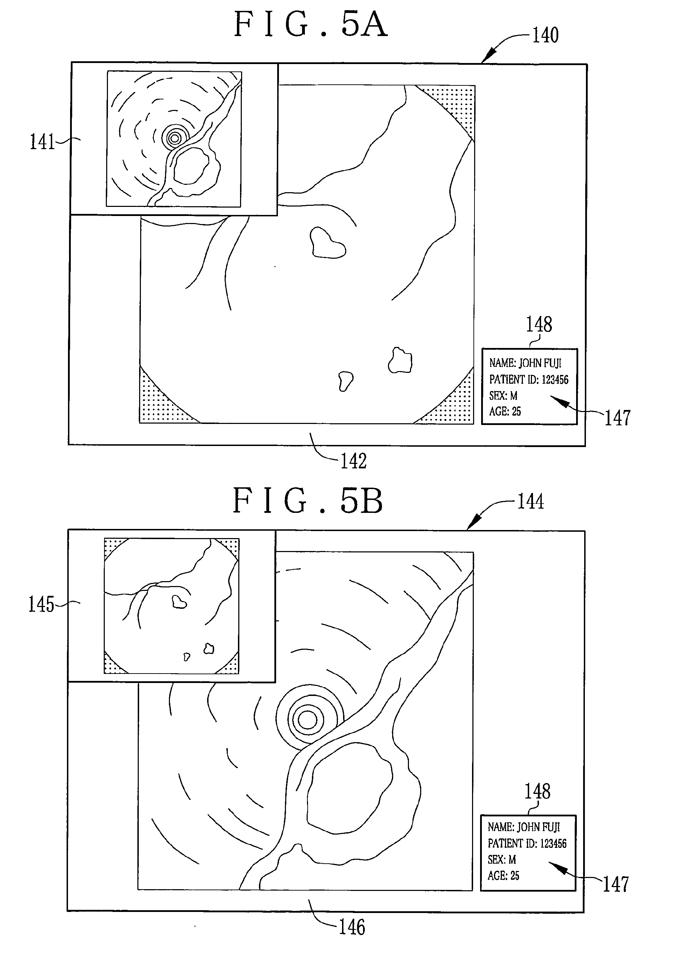

In Fig. 1, an ultrasonic diagnosis system 2 includes an ultrasonic endoscope 10, an ultrasonic processor 12, an endoscope processor 14, a light source unit 16, a monitor display panel 18 and a movable cart 20. The ultrasonic processor 12 or control processor creates an ultrasonic image 146 of Fig. 5. The endoscope processor 14 creates an endoscopic image 142. The light source unit 16 emits illumination light and applies the light to an object in a human cavity of a patient's body through the ultrasonic endoscope 10. The monitor display panel 18 displays the endoscopic or ultrasonic image 142 or 146. The movable cart 20 supports those devices.

The ultrasonic endoscope 10 includes an insertion tube 22, a handle 23 and a universal cable 24. The insertion tube 22 is entered in a patient's body. The handle 23 is manually operable, from which the insertion tube 22 extends. The universal cable 24 extends from the handle 23. A distal end of the universal cable 24 has a contact plug 26, an endoscope plug 27 and a light source plug 28. The contact plug 26 is connectable with the ultrasonic processor 12. The endoscope plug 27 is connectable with the endoscope processor 14. The light source plug 28 is connectable with the light source unit 16. The contact plug 26, the endoscope plug 27 and the light source plug 28 are used in connection of the ultrasonic endoscope 10 with respectively the ultrasonic processor 12, the endoscope processor 14 and the light source unit 16.

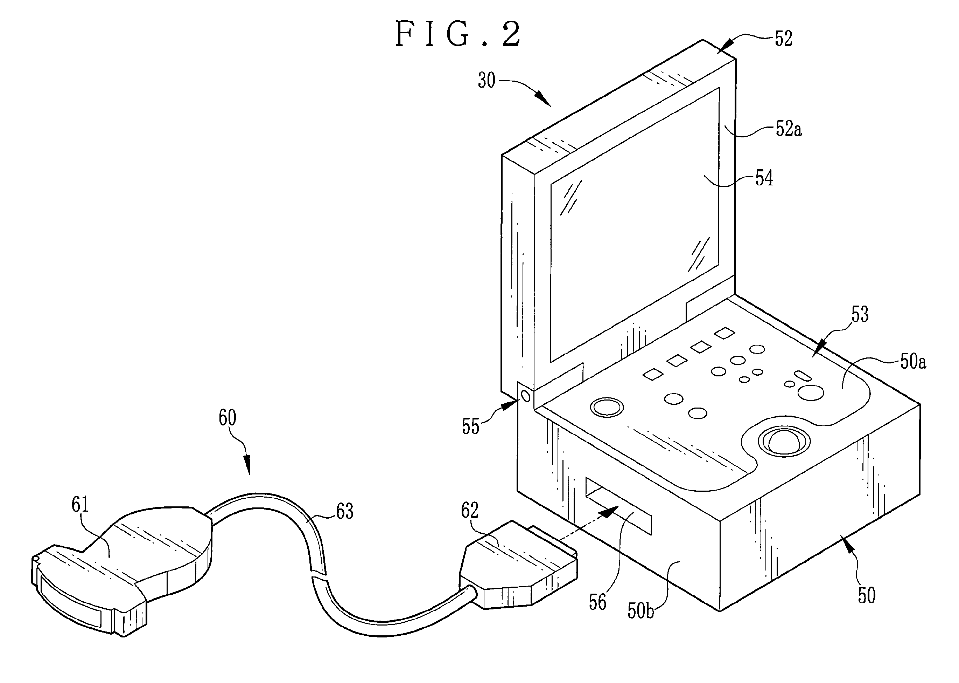

The ultrasonic processor 12 includes a portable ultrasonic diagnosis device 30 and an adapter 32. The ultrasonic diagnosis device 30 in an ultrasonic diagnosis apparatus operates for extracorporeal device diagnosis. The adapter 32 enables combined use of the ultrasonic diagnosis device 30 with the ultrasonic endoscope 10. There is a difference in the interface between a first ultrasonic probe 60 for extracorporeal device diagnosis of Fig. 2 and the ultrasonic endoscope 10. The ultrasonic endoscope 10 cannot be connected directly with the ultrasonic diagnosis device 30.

The adapter 32 is an interface for coupling the ultrasonic endoscope 10 with the ultrasonic diagnosis device 30. Also, the adapter 32 operates for additional functions auxiliary to the ultrasonic diagnosis device 30, so that performance of the ultrasonic processor 12 can be enhanced.

The ultrasonic diagnosis system 2 is thus effective in cost saving of instruments in a hospital for examination, because the ultrasonic diagnosis device 30 for extracorporeal device diagnosis can be used with the ultrasonic endoscope 10 and utilized as a single component for multiple purposes.

The movable cart 20 includes a top tray 40, tiers 41 and 42, a hanger 43 and a post 44. The top tray 40 is used for supporting various tools and chemicals for use in examination of a patient's body. The tiers 41 and 42 support various units such as the ultrasonic processor 12, the endoscope processor 14, the light source unit 16 and the like. The hanger 43 suspends the ultrasonic endoscope 10. The post 44 supports the monitor display panel 18. The top tray 40 is supported on the top end of the movable cart 20, and is removable from the movable cart 20 to facilitate washing, parts exchange or maintenance at the time of pollution with chemicals or other unrelated fluid. The post 44 is nearly cylindrical, and is kept rotatable by a rotating mechanism for directing the monitor display panel 18 in any direction, and is kept movable up and down by a height adjusting mechanism.

The movable cart 20 includes a base 45 of a flat plate shape, and casters 46 disposed on corners of a lower surface of the base 45. The elements of the ultrasonic diagnosis system 2 set on the movable cart 20 can be placed to a desired position in an examination room according to a user's intention by use of the casters 46.

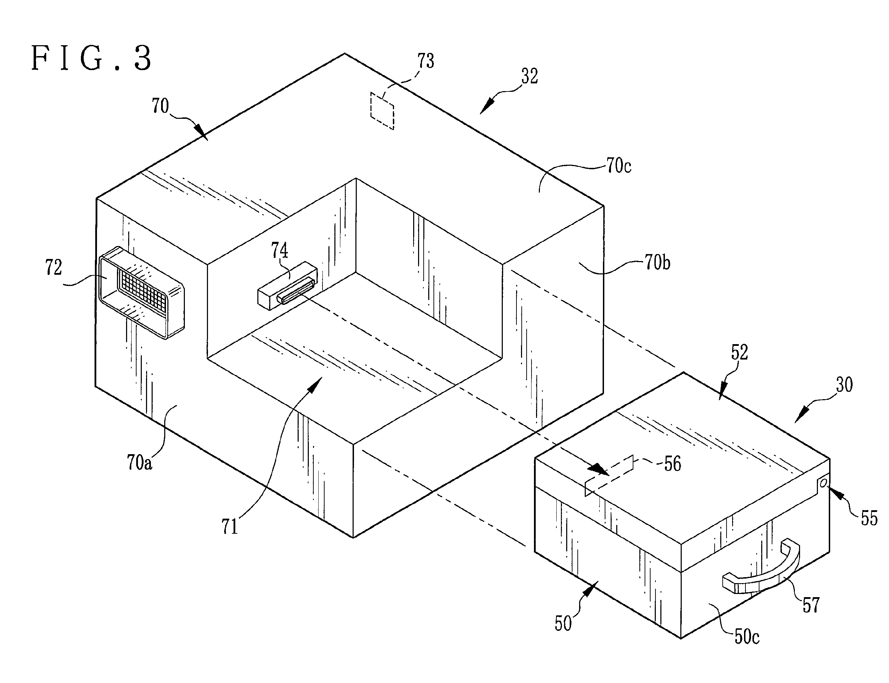

In Fig. 2, the ultrasonic diagnosis device 30 includes a body 50 and a cover 52. The body 50 has a box shape. The cover 52 is a plate of a flat shape. An upper face 50a of the body 50 is provided with plural buttons, trackballs and the like. An input panel 53 is operable to input various command signals to the ultrasonic diagnosis device 30. A display panel 54 is secured to an inner surface 52a of the cover 52 and displays the ultrasonic image 146 and various menus. A hinge 55 keeps the cover 52 pivotally movable on the body 50 between an open position of Fig. 2 to uncover the input panel 53 and the display panel 54 and a closed position of Fig. 3 to cover those.

A connection terminal 56 for the first ultrasonic probe 60 of extracorporeal use is disposed on a first lateral surface 50b of the body 50, and adapted to connection of the first ultrasonic probe 60 in a removable manner. A grip 57 of Fig. 3 is disposed on a right lateral surface 50c of the body 50, and is manually grasped for carrying the ultrasonic diagnosis device 30 or mounting the same on the adapter 32.

The first ultrasonic probe 60 in the ultrasonic diagnosis apparatus includes a scan head 61, a probe interface 62, and a cable 63. The scan head 61 emits ultrasonic waves and receives echo ultrasonically reflected by an object. The cable 63 extends between the scan head 61 and the probe interface 62. In the ultrasonic diagnosis device 30, the connection terminal 56 of the body 50 is connectable with the probe interface 62 of the first ultrasonic probe 60.

The ultrasonic diagnosis device 30 controls the first ultrasonic probe 60 by means of the connection terminal 56 and the probe interface 62. The first ultrasonic probe 60 is controlled by the ultrasonic diagnosis device 30 and emits and receives ultrasonic waves, and converts echo of the reflection piezoelectrically into a detection signal, which is input to the ultrasonic diagnosis device 30. The ultrasonic diagnosis device 30, in response to the detection signal from the first ultrasonic probe 60, creates the ultrasonic image 146, which is displayed on the display panel 54. Thus, extracorporeal device diagnosis is carried out by the first ultrasonic probe 60 and the ultrasonic diagnosis device 30.

In Fig. 3, a casing 70 of the adapter 32 has a box shape easy to mount on the movable cart 20. A holder chamber 71 is formed in the casing 70 for receiving entry of the ultrasonic diagnosis device 30. A front face 70a, a right lateral surface 70b and an upper lateral surface 70c of the casing 70 are recessed to define the holder chamber 71 according to the shape of the ultrasonic diagnosis device 30. Also, an in-vivo probe connector 72 is disposed in the front face 70a of the casing 70 for connection of the contact plug 26 of the ultrasonic endoscope 10 in a removable manner. A processor connector 73 is disposed in a rear face of the casing 70 for connection with the endoscope processor 14.

A diagnosis device connector 74 is disposed in the holder chamber 71 for connection with the ultrasonic diagnosis device 30. A shape of the diagnosis device connector 74 is equal to that of the probe interface 62 of the first ultrasonic probe 60. When the ultrasonic diagnosis device 30 is set inside the holder chamber 71, the diagnosis device connector 74 becomes connected with the connection terminal 56.

The adapter 32 becomes electrically connected with the ultrasonic diagnosis device 30 through the diagnosis device connector 74 and the connection terminal 56. Also, the adapter 32 becomes electrically connected with the ultrasonic endoscope 10 through the probe connector 72 and the contact plug 26. Thus, the adapter 32 couples the ultrasonic endoscope 10 with the ultrasonic diagnosis device 30 for inputs and outputs.

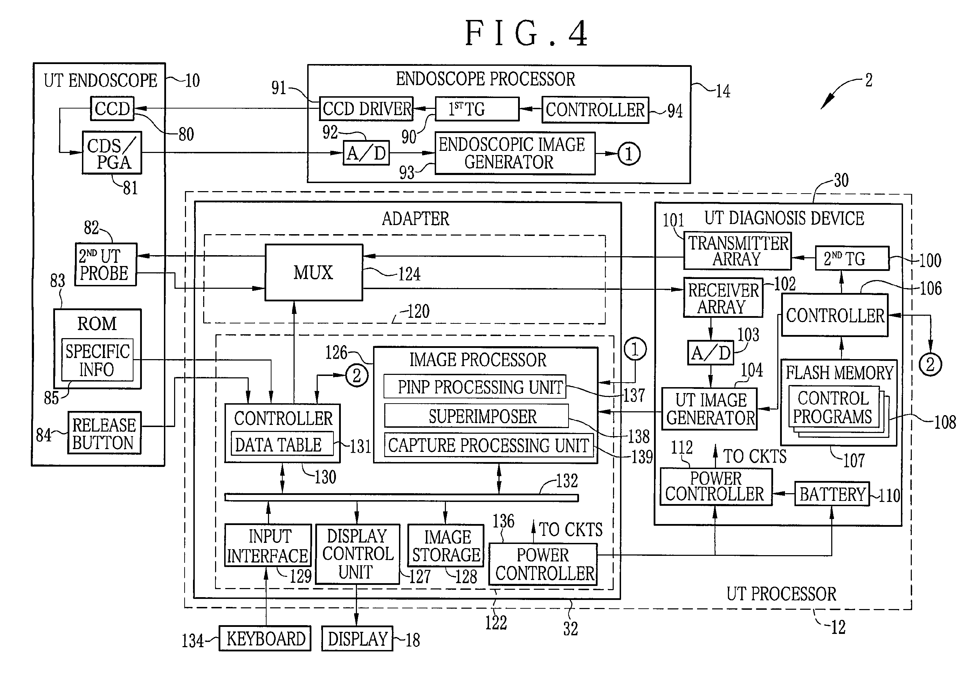

In Fig. 4, the ultrasonic endoscope 10 includes a CCD 80, a correlated double sampling/programmable gain amplifier (CDS/PGA) 81, a second ultrasonic probe 82 or ultrasonic transducer array, a ROM 83 and a release button 84. The CCD 80 and the second ultrasonic probe 82 are disposed at the end of the insertion tube 22. The CCD 80 picks up an object image viewed through the imaging window, and generates an image signal of the object image. The CDS/PGA 81 eliminates electric noise from the image signal of the CCD 80 and amplifies the same.

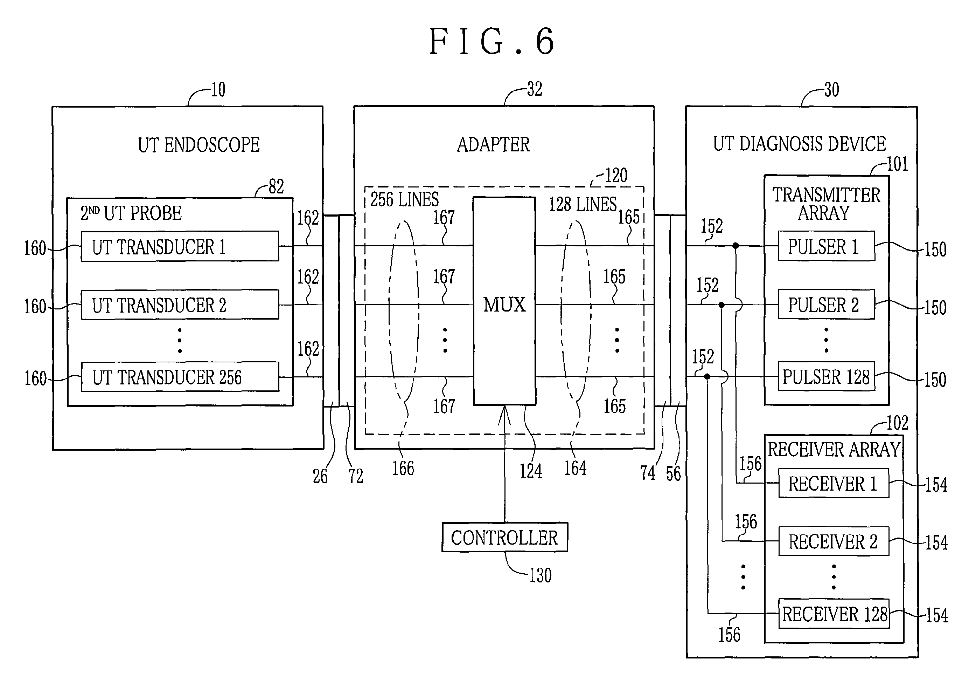

256 ultrasonic transducers 160 are arranged in the second ultrasonic probe 82 in a two dimensional manner in Fig. 6. The ultrasonic transducers 160 emit ultrasonic waves. Also, the ultrasonic transducers 160 receive ultrasonic waves reflected by an object in a body, and convert the same piezoelectrically to generate a detection signal according to the reflected ultrasonic waves. Specific information 85 is stored in the ROM 83 for discerning a type of the ultrasonic endoscope 10. Examples of the specific information 85 include information of a type or lot number of the ultrasonic endoscope 10 or the like for discerning the type.

The release button 84 is operable to record the endoscopic or ultrasonic image 142 or 146 as a still image while a motion image is displayed on the monitor display panel 18. The release button 84 is disposed on the handle 23. When the release button 84 is depressed, a still image is recorded, to store the endoscopic or ultrasonic image 142 or 146 in a data file of the still image in a digital form. A physician or operator in a hospital depresses the release button 84 upon finding a lesion or object of interest, to create the data file of the still image.

The endoscope processor 14 includes a first timing generator 90 for CCD, a CCD driver 91, an A/D converter 92, an endoscopic image generator 93 and a controller 94. The controller 94 controls various circuit elements in the endoscope processor 14.

The first timing generator 90 is controlled by the controller 94 and inputs a timing signal or clock pulse to the CCD driver 91. In response, the CCD driver 91 inputs a drive signal to the CCD 80, and controls timing of reading the stored charge of the CCD 80, its electronic shutter speed, and the like.

The A/D converter 92 converts the image signal of the analog form from the CDS/PGA 81 into image data of a digital form. The endoscopic image generator 93 processes the digital image data from the A/D converter 92 in the image processing of various functions, and creates the endoscopic image 142. The endoscopic image generator 93 outputs information of the endoscopic image 142 to the adapter 32.

Also, it is possible to display the endoscopic image 142 on the monitor display panel 18 directly by connecting this with the endoscope processor 14. The endoscope processor 14 can be used in the multiple use, because usable for endoscopic examination with an endoscope as well as in-vivo diagnosis with the ultrasonic diagnosis system 2. The cost saving for instruments in the hospital is possible by adapting the endoscope processor 14 in the ultrasonic diagnosis system 2.

The ultrasonic diagnosis device 30 includes a second timing generator 100 for ultrasonic imaging, a transmitter array 101, a receiver array 102, an A/D converter 103, an ultrasonic image generator 104, a controller 106, and a flash memory 107. A plurality of control programs 108 are stored in the flash memory 107 for controlling the ultrasonic diagnosis device 30. The controller 106 reads a specific one of the control programs 108 from the flash memory 107, and controls various circuits in the ultrasonic diagnosis device 30 entirely by successively running the control program 108.

The second timing generator 100 is controlled by the controller 106, and supplies the transmitter array 101 with a drive pulse. The transmitter array 101 generates an excitation pulse or pulse voltage for the ultrasonic transducers 160 in the second ultrasonic probe 82 to emit ultrasonic waves according to the drive pulse from the second timing generator 100. The receiver array 102 receives a detection signal from the second ultrasonic probe 82 upon receiving the reflected ultrasonic waves, and outputs the detection signal to the A/D converter 103.

The A/D converter 103 converts the detection signal of the analog form from the receiver array 102 into digital image data. The ultrasonic image generator 104 processes the image data from the A/D converter 103 in image processing of various functions, to create the ultrasonic image 146. If the diagnosis device connector 74 is connected with the connection terminal 56, the ultrasonic image generator 104 outputs the ultrasonic image 146 to the adapter 32. If the probe interface 62 is connected with the connection terminal 56, the ultrasonic image generator 104 outputs the ultrasonic image 146 to the display panel 54.

Furthermore, the ultrasonic diagnosis device 30 has a battery 110 and a power controller 112, which is supplied with power by the battery 110, converts the voltage and the like, and powers various elements in the ultrasonic diagnosis device 30. The ultrasonic diagnosis device 30 can carry out the extracorporeal device diagnosis with the first ultrasonic probe 60 without connection of a power source cable or the like. Examples of the battery 110 are a nickel cadmium battery, lithium ion battery, and other secondary batteries.

The adapter 32 includes an input/output device 120 and a control device 122 or auxiliary device for auxiliary performance. The input/output device 120 provides access between the ultrasonic endoscope 10 in connection with the probe connector 72 and the ultrasonic diagnosis device 30 in connection with the diagnosis device connector 74. The control device 122 carries out various functions auxiliary to the ultrasonic diagnosis device 30. The input/output device 120 includes a multiplexer 124 (MUX). The control device 122 includes an image processor 126, a display control unit 127, an image storage 128, an input interface 129, and a controller 130. There is a data bus 132 where various elements of the control device 122 are connected together.

The controller 130 is connected with the multiplexer 124. The multiplexer 124 is controlled by the controller 130, and selectively changes over the connection line between the second ultrasonic probe 82 or ultrasonic transducer array and each of the transmitter array 101 and the receiver array 102.

To the image processor 126 are connected the endoscopic image generator 93 of the endoscope processor 14 and the ultrasonic image generator 104 of the ultrasonic diagnosis device 30. The image processor 126 processes the endoscopic image 142 from the endoscopic image generator 93 in image processing, and processes the ultrasonic image 146 from the ultrasonic image generator 104 in image processing.

The display control unit 127 receives the endoscopic or ultrasonic image 142 or 146 after the image processing in the image processor 126, and converts this to a video signal (component signal, composite signal or the like) in compliance with the format of the monitor display panel 18. Thus, the endoscopic and ultrasonic images 142 and 146 are displayed on the monitor display panel 18. The image storage 128 is constituted by an HDD, semiconductor memory or the like, and stores an image file of a still image obtained upon depression of the release button 84.

A keyboard 134 is connected with the input interface 129 for inputting alphanumeric information, command signals and the like. The input interface 129 converts the inputs from the keyboard 134 into a signal of a format which circuit elements in the adapter 32 can treat. The keyboard 134 is supported in a position directly under the top tray 40 of the movable cart 20 in a slidable manner, and moves between a first position contained in the movable cart 20 under the top tray 40 and a second position protruding in front of the movable cart 20 to uncover its keys.

The multiplexer 124 and the data bus 132 are connected with the controller 130. Also, the ROM 83 and the release button 84 are connected with the controller 130 by the contact plug 26 and the probe connector 72. Also, the controller 130 is connected to the controller 106 of the ultrasonic diagnosis device 30 by the diagnosis device connector 74 and the connection terminal 56.

A power controller 136 as battery charger is incorporated in the adapter 32. The power controller 136 is supplied with power of a commercial power source through a power source cable (not shown), converts the power in the AC/DC conversion and voltage conversion, and supplies circuits in the adapter 32 with the converted power. Also, the power controller 136 becomes connected with the power controller 112 of the ultrasonic diagnosis device 30 and the battery 110 by the diagnosis device connector 74 and the connection terminal 56. When the holder chamber 71 is loaded with the ultrasonic diagnosis device 30, the power controller 136 supplies the battery 110 with power for charging. At the same time, the power controller 136 supplies the power controller 112 with power. The power controller 112 supplies various elements in the ultrasonic diagnosis device 30 with power by use of power from the power controller 136 without use of the battery 110.

The image processor 126 includes a picture-in-picture (PinP) processing unit 137, a superimposer 138, and a capture processing unit 139. The picture-in-picture processing unit 137 processes images by disposing a small image within an image for observation of plural images in one image frame. The superimposer 138 superimposes alphanumeric information 147 or a patterned line 148 on one image. The capture processing unit 139 records a still image processed in the picture-in-picture processing or the combining step of the alphanumeric information 147 in response to depression of the release button 84.

The picture-in-picture processing unit 137 operates in response to signals input by use of the keyboard 134, and creates a first combined image 140 of Fig. 5A by forming a size reduction ultrasonic image 141 or low resolution image with the endoscopic image 142. Also, the picture-in-picture processing unit 137 creates a second combined image 144 of Fig. 5B by forming a size reduction endoscopic image 145 or low resolution image with the ultrasonic image 146.

The superimposer 138 responds to input signals from the keyboard 134, and combines the alphanumeric information 147 or the patterned line 148 with the endoscopic or ultrasonic image 142 or 146 or the first or second combined image 140 or 144 from the picture-in-picture processing unit 137.

Selective display on the monitor display panel 18 among the endoscopic and ultrasonic images 142 and 146 and the first and second combined images 140 and 144 is determined according to an input signal from the keyboard 134. If display of the first or second combined image 140 or 144 is designated with the keyboard 134, the image processor 126 drives the picture-in-picture processing unit 137 to create the first or second combined image 140 or 144. If display of the alphanumeric information 147 or the patterned line 148 is designated with the keyboard 134, the image processor 126 drives the superimposer 138 to superimpose the alphanumeric information 147 or the patterned line 148 on an image designated for display on the monitor display panel 18.

The controller 130 instructs the capture processing unit 139 to record a still image upon depression of the release button 84. The capture processing unit 139 in response to this records an image as output to the monitor display panel 18 in a form of a still image, and creates a data file of the still image. The capture processing unit 139 writes the data file to the image storage 128.

In Fig. 6, 128 pulsers 150 are included in the transmitter array 101 of the ultrasonic diagnosis device 30, and output an excitation pulse according to a drive pulse from the second timing generator 100. There is a signal line 152. A first end of the signal line 152 is connected with each one of the pulsers 150. A second end of the signal line 152 is connected with the connection terminal 56. 128 receivers 154 are included in the receiver array 102 for receiving a detection signal from the second ultrasonic probe 82 or ultrasonic transducer array. The receivers 154 are associated with the pulsers 150 one by one. There is a signal line 156. A first end of the signal line 156 is connected with each one of the receivers 154. A second end of the signal line 156 is connected with the signal line 152 of a corresponding one of the pulsers 150.

256 ultrasonic transducers 160 are arranged in the second ultrasonic probe 82 of the ultrasonic endoscope 10 as described above. A signal line 162 is associated with each of the ultrasonic transducers 160. A first end of the signal line 162 is connected with the ultrasonic transducers 160. A second end of the signal line 162 is connected with the contact plug 26.

The input/output device 120 in the adapter 32 includes the multiplexer 124, a first signal line group 164 and a second signal line group 166. 128 signal lines 165 constitute the first signal line group 164 and correspond to the pulsers 150. A first end of the signal lines 165 is connected with the multiplexer 124. A second end of the signal lines 165 is connected with the diagnosis device connector 74. Each of the signal lines 165 is connected with the signal line 152 in the ultrasonic diagnosis device 30 through the diagnosis device connector 74 and the connection terminal 56.

256 signal lines 167 are included in the second signal line group 166 and associated with the ultrasonic transducers 160. A first end of the signal lines 167 in the second signal line group 166 is connected with the multiplexer 124. A second end of the signal lines 167 is connected with the probe connector 72. Each of the signal lines 167 is connected with the signal line 162 of the ultrasonic endoscope 10 by use of the probe connector 72 and the contact plug 26.

The pulsers 150 must be connected with respectively the ultrasonic transducers 160 to obtain the ultrasonic image 146 by driving the second ultrasonic probe 82. As the number of the pulsers 150 in the transmitter array 101 is smaller than the number of the ultrasonic transducers 160 in the second ultrasonic probe 82, some of the ultrasonic transducers 160 cannot be driven by direct connection of those with the pulsers 150. The ultrasonic image 146 of a normal form cannot be retrieved.

The multiplexer 124 to solve such a problem changes over the connection line between the first and second signal line groups 164 and 166 to change over the ultrasonic transducers 160 to be driven by the pulsers 150. Thus, the ultrasonic image 146 can be retrieved normally even if the number of the pulsers 150 is small.

Changeover of the line connection of the multiplexer 124 is controlled by the controller 130. A start button (not shown) is depressed to start display of the ultrasonic image 146. In response to this, the controller 130 accesses the ROM 83 of the ultrasonic endoscope 10 to read the specific information 85. The controller 130 writes the specific information 85 to a working memory, and transmits the same to the controller 106 of the ultrasonic diagnosis device 30.

The method of driving the pulsers 150 and method of creating the ultrasonic image 146 in the ultrasonic image generator 104 are different according to various factors in a driving condition of the ultrasonic transducers 160, such as the number of the pulsers 150, the number of the ultrasonic transducers 160, a difference between those numbers, and a size and angle of the ultrasonic transducers 160. The control programs 108 in the flash memory 107 are predetermined for respectively the driving conditions. The driving conditions of the ultrasonic transducers 160 are predetermined for the types of the ultrasonic endoscope 10. The control programs 108 are assigned with type information of the types or other metadata, and associated with the types of the ultrasonic endoscope 10.

The controller 106, upon receiving the specific information 85 from the controller 130 of the adapter 32, refers to the flash memory 107 at the location of the specific information 85, and reads one of the control programs 108 from the flash memory 107 according to one of the types of the ultrasonic endoscope 10 in connection. The second timing generator 100 and the ultrasonic image generator 104 are controlled according to the control program 108 in a manner suitable for the type of the ultrasonic endoscope 10. Also, the controller 106, upon starting the control according to the control program 108, sends a sync signal to the controller 130 of the adapter 32.

The controller 130 has an internal memory, which stores a data table 131 as data of a relationship between types of the ultrasonic endoscope 10 and driving conditions of the ultrasonic transducers 160. Examples of the driving conditions are the number, size, angle of orientation and the like of the ultrasonic transducers 160. The controller 130, in response to a sync signal from the controller 106, refers to the data table 131 according to the specific information 85 in the working memory, and reads one of the driving conditions from the data table 131 according to the type of the ultrasonic endoscope 10. Then the controller 130 controls the changeover of the connection line of the multiplexer 124. The ultrasonic transducers 160 in the second ultrasonic probe 82 are driven to create the ultrasonic image 146.

The operation of the ultrasonic diagnosis system 2 is described now. In the ultrasonic diagnosis device 30, the first ultrasonic probe 60 is connected with the connection terminal 56 and the cover 52 is set in the open position to uncover the input panel 53 and the display panel 54, so that extracorporeal device diagnosis is possible. In contrast, the ultrasonic diagnosis system 2 is constituted by securing the ultrasonic diagnosis device 30 to the adapter 32 with the cover 52 set in the closed position, so that in-vivo diagnosis is possible. In the present embodiment, the ultrasonic diagnosis device 30 as a single component can operate both for in-vivo diagnosis and for extracorporeal device diagnosis. The cost saving for instruments in a hospital is possible.

A physician or operator prepares for in-vivo diagnosis by use of the ultrasonic diagnosis system 2. Various elements of the ultrasonic diagnosis system 2 are set up as illustrated in Fig. 1. Tools and chemicals are placed on the top tray 40 of the movable cart 20. As the casing 70 of the adapter 32 is shaped like a box, the adapter 32 can be mounted easily on the movable cart 20. The battery 110 of the ultrasonic diagnosis device 30 is supplied with power by the power controller 136 in the adapter 32 when the ultrasonic diagnosis device 30 is mounted, and can be charged.

The physician, upon becoming ready for the examination, depresses a start button (not shown) of the endoscope processor 14 for input a command signal for the start. The controller 94, in response to the command signal, controls the first timing generator 90 to start driving the CCD 80 with the CCD driver 91. The CCD 80 driven by the CCD driver 91 picks up an image of the object through an imaging window, and creates an image signal according to an object image. The image signal is processed by the CDS/PGA 81 for noise reduction and amplification, and is input to the A/D converter 92 and converted into image data of a digital form. The image data is sent to the endoscopic image generator 93. The endoscopic image generator 93 processes the image data in image processing of various functions, and obtains the endoscopic image 142.

The endoscopic image 142 being created is input to the adapter 32, processed by the image processor 126 and displayed on the monitor display panel 18 by the display control unit 127. The physician, upon viewing the endoscopic image 142, enters the insertion tube 22 of the ultrasonic endoscope 10 in a human cavity or gastrointestinal tract of a patient's body, and starts observation. If he or she wishes to observe the tissue surface of an object in the body, specifically if a lesion is found, then he or she depresses a start button (not shown) in the adapter 32, to start display of the ultrasonic image 146 in the ultrasonic processor 12.

When a start button is depressed to generate a command signal for display of the ultrasonic image 146, the controller 130 in the adapter 32 accesses the ROM 83 of the ultrasonic endoscope 10 to read the specific information 85 from the ROM 83. The controller 130 writes the specific information 85 to its working memory, and transmits the same to the controller 106 of the ultrasonic diagnosis device 30.

The controller 106, upon receiving the specific information 85 from the controller 130 of the adapter 32, refers to the flash memory 107, and reads one of the control programs 108 from the flash memory 107 according to the type of the ultrasonic endoscope 10 in connection. The controller 106 starts controlling the second timing generator 100 and the ultrasonic image generator 104 according to the control program 108. At the same time, the controller 106 sends a sync signal for the start to the controller 130 of the adapter 32.

The second timing generator 100 is controlled by the controller 106 and sends a drive pulse to respectively the pulsers 150 of the transmitter array 101. The pulsers 150 output an excitation pulse according to the drive pulse. The excitation pulse from the pulsers 150 is input to the multiplexer 124. The controller 130, upon receiving a sync signal from the controller 106, refers to the data table 131 according to the specific information 85 read from a working memory, and retrieves information of a driving condition from the data table 131 according to the type of the ultrasonic endoscope 10. Then the multiplexer 124 is controlled by the retrieved driving condition, to change over the line connection between the first and second signal line groups 164 and 166, selectively to designate the ultrasonic transducers 160 driven by the pulsers 150. The excitation pulse output by the pulsers 150 is sent through the multiplexer 124 to respectively the ultrasonic transducers 160.

The ultrasonic transducers 160 emit ultrasonic waves in response to the excitation pulse generated by the pulsers 150, and also receive the ultrasonic waves reflected by the object. The reflected ultrasonic waves are piezoelectrically converted to generate a detection signal. The receiver array 102 is supplied with the detection signal by the multiplexer 124. An associated one of the receivers 154 receives the detection signal in correspondence with the pulsers 150.

The receivers 154 output the received detection signal to the A/D converter 103. The A/D converter 103 converts the detection signal of the analog form from the receivers 154 into digital image data, which is input to the ultrasonic image generator 104. The ultrasonic image generator 104 processes the digital image data from the A/D converter 103 in the image processing of various functions, to create the ultrasonic image 146.

The ultrasonic image 146 is input to the adapter 32, processed by the image processor 126 and displayed on the monitor display panel 18 by the display control unit 127. As the multiplexer 124 is provided in the input/output device 120 in the adapter 32 for selective driving of the ultrasonic transducers 160 by use of the pulsers 150, the ultrasonic image 146 can be obtained in a normal form even when the pulsers 150 in the transmitter array 101 are fewer than the ultrasonic transducers 160 in the second ultrasonic probe 82.

A form of the ultrasonic image 146 for display on the monitor display panel 18 can be changed over by inputting a signal with the keyboard 134 in operation of the ultrasonic processor 12. When display of the endoscopic or ultrasonic image 142 or 146 is designated, the image processor 126 in the adapter 32 causes the display control unit 127 to drive the monitor display panel 18 for displaying the designated image. When the first combined image 140 is designated, the image processor 126 causes the picture-in-picture processing unit 137 to process the endoscopic and ultrasonic images 142 and 146 and create the first combined image 140. The display control unit 127 is supplied with information of the first combined image 140 and displays this on the monitor display panel 18. Similarly, when the second combined image 144 is designated, the image processor 126 causes the picture-in-picture processing unit 137 to create the second combined image 144, which is displayed on the monitor display panel 18.

It is possible to superimpose the alphanumeric information 147 or the patterned line 148 on the images 140, 142, 144 and 146 by use of an input signal from the keyboard 134 to the superimposer 138 to display the images 140, 142, 144 and 146 on the monitor display panel 18.

Upon depressing the release button 84 while the images 140, 142, 144 and 146 are displayed on the monitor display panel 18, recording of the still images 140, 142, 144 and 146 is carried out according to a signal from the capture processing unit 139. An image file of data according to the images 140, 142, 144 and 146 is stored in the image storage 128.

In the embodiment, the control device 122 or auxiliary device operates additionally to the ultrasonic diagnosis device 30. It is possible to simplify the structure of the ultrasonic diagnosis device 30 and to maintain the general-purpose property of examination with the ultrasonic diagnosis device 30 having a limited structure. The in-vivo diagnosis can be simple without complicated steps, because operation of the ultrasonic diagnosis device 30 is assisted by performance of the control device 122. As the ultrasonic diagnosis device 30 is relatively inexpensive, a great number of hospitals can have one. There is no need of adapting a diagnosis apparatus for in-vivo diagnosis with a comparatively high cost, because the use of the adapter 32 is effective in combination with the ultrasonic diagnosis device 30 and a computer program of a selective use.

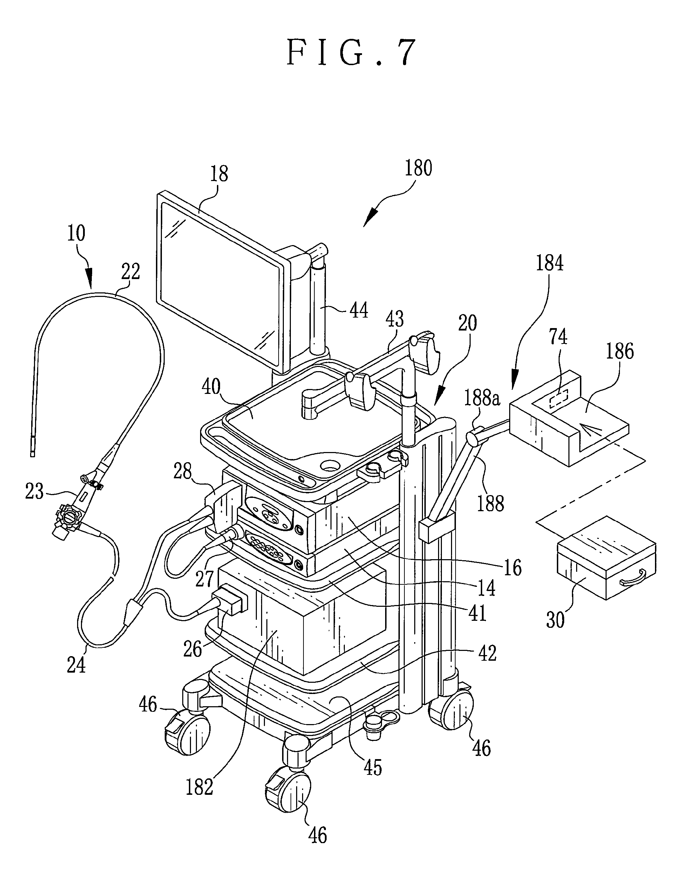

In the above embodiment, the holder chamber 71 is disposed in the casing 70. Another preferred ultrasonic diagnosis system 180 is illustrated in Fig. 7, in which a holder chamber is separate from its casing. In the ultrasonic diagnosis system 180, an adapter includes a casing 182 and a base dock 184. The casing 182 has the probe connector 72 for connection of the contact plug 26 of the ultrasonic endoscope 10. The base dock 184 includes a holder chamber 186 and an arm 188. The holder chamber 186 contains the ultrasonic diagnosis device 30. The arm 188 supports the holder chamber 186.

The holder chamber 186 includes the diagnosis device connector 74 for connection with the ultrasonic diagnosis device 30. The arm 188 is secured to the movable cart 20. There is a movable portion 188a together with a rotating mechanism in the arm 188, to enable adjustment of a height and direction of the ultrasonic diagnosis device 30 in the holder chamber 186.

The base dock 184 is connected electrically with the casing 182 by a cable (not shown). Thus, the ultrasonic diagnosis device 30 in the holder chamber 186 becomes connected with the ultrasonic endoscope 10 by the base dock 184 and the casing 182. Loading and unloading of the ultrasonic diagnosis device 30 can be easier according to separate construction of the casing 182 and the holder chamber 186 for the adapter. In containing the ultrasonic diagnosis device 30 in the base dock 184, it is possible to display the endoscopic image 142 on the monitor display panel 18 and the ultrasonic image 146 on the display panel 54 of the ultrasonic diagnosis device 30, so that the endoscopic and ultrasonic images 142 and 146 can be observed at one time. Although the casing 182 without the holder chamber 71 is illustrated in Fig. 7, it is possible to form the holder chamber 71 and to connect the ultrasonic diagnosis device 30 by use of a selected one of the holder chambers 71 and 186.

An ultrasonic probe of the invention, in place of the ultrasonic endoscope 10, may have any of other structures, such as an ultrasonic probe for insertion through a forceps channel in an ultrasonic endoscope.

Various functions can be added in the control device 122 or auxiliary device. In the embodiment, functions in the control device 122 are display of images with the display control unit 127, input of command signals with the input interface 129, the PinP processing with the picture-in-picture processing unit 137, superimposition of the alphanumeric information 147 on an image with the superimposer 138, capture of an image with the capture processing unit 139, storage of images with the image storage 128, and charging of the battery 110 with the power controller 136. Also, other functions may be added.

Note that images created by the picture-in-picture processing unit 137 are not limited to the combined image 140 or 144 according to the above embodiment. For example, a combined image created by the picture-in-picture processing unit 137 may be constituted by a still image and a motion image which has a small size and combined with the still image, specifically when freezing of an endoscopic or ultrasonic image is instructed.

In the above embodiment, an image file of still images is retrieved by the capture processing unit 139. However, an image file of a motion image can be retrieved. In the above embodiment, the keyboard 134 is used as input interface. However, an input interface may be any of known input devices, such as buttons, switches, mouse, pads, joystick and the like.

In the above embodiment, the controller 130 of the adapter 32 controls the changeover in the multiplexer 124. However, the controller 106 of the ultrasonic diagnosis device 30 may control the changeover in the multiplexer 124. Furthermore, the line connection may be changed over by circuit elements other than the multiplexer 124, for example, a semiconductor switch, mechanical relay or the like known in the art.

In the above embodiment, the data table 131 is stored in an internal memory of the controller 130. However, the data table 131 may be stored in a semiconductor memory in connection with the data bus 132, the flash memory 107 of the ultrasonic diagnosis device 30, or a server connected by use of the LAN or other networks. In the above embodiment, the specific information 85 is read for discerning a type of the ultrasonic endoscope 10. However, it is possible to read specific information for discerning a type of the second ultrasonic probe 82. Furthermore, information of the driving condition of the ultrasonic transducers 160 can be stored and read. This makes it unnecessary to store the data table 131.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An adapter comprising:
a holder chamber (71, 186) for holding an ultrasonic diagnosis device (30) adapted to diagnosis according to a signal from a first ultrasonic probe (60) for extracorporeal use;
a diagnosis device connector (74) for connection with said ultrasonic diagnosis device;
a probe connector (72) for connection with a second ultrasonic probe (10, 82) for in-vivo diagnosis to detect an object;
an input/output device (120) for inputting and/or outputting between said ultrasonic diagnosis device and said second ultrasonic probe through said probe connector and said diagnosis device connector;
an auxiliary device (122) for performance auxiliary to said ultrasonic diagnosis device.

2. An adapter as defined in claim 1, wherein said diagnosis device connector is connectable with a connection terminal of said ultrasonic diagnosis device for said first ultrasonic probe.

3. An adapter as defined in claim 2, wherein said holder chamber is shaped in a box-shaped recessed form, and said diagnosis device connector is positioned on an inner surface of said holder chamber.

4. An adapter as defined in claim 3, wherein said ultrasonic diagnosis device includes a lower surface, a first lateral surface oriented erectly from said lower surface, and a second lateral surface oriented transversely with said first lateral surface;
said holder chamber includes a support surface for supporting said lower surface, a first inner surface oriented erectly from said support surface and opposed to said first lateral surface, and a second inner surface oriented erectly from said support surface and opposed to said second lateral surface;
said connection terminal is disposed on said first lateral surface, and said diagnosis device connector is disposed on said first inner surface.

5. An adapter as defined in claim 1, further comprising a processor connector for connection with an endoscope processor for receiving a signal from an endoscope to create an endoscopic image.

6. An adapter as defined in claim 5, wherein said second ultrasonic probe is disposed on said endoscope;
said endoscope includes an image sensor for picking up said object and outputting a signal to said endoscope processor.

7. An adapter as defined in claim 5, wherein said auxiliary device includes a display control unit for control of displaying at least one of an ultrasonic image from said ultrasonic diagnosis device and said endoscopic image from said endoscope processor.

8. An adapter as defined in claim 5, wherein said auxiliary device includes a picture-in-picture processing unit for picture-in-picture processing of an ultrasonic image or said endoscopic image.

9. An adapter as defined in claim 5, wherein said auxiliary device includes a superimposer for control of superimposing alphanumerical information or a patterned line on an ultrasonic image or said endoscopic image.

10. An adapter as defined in claim 1, further comprising an input interface for inputting a command signal for operation.

11. An adapter as defined in claim 1, further comprising a battery charger for charging a battery contained in said ultrasonic diagnosis device.

12. An adapter as defined in claim 1, further comprising a casing for containing said input/output device and said auxiliary device;
wherein said holder chamber is formed with said casing.

13. An adapter as defined in claim 1, further comprising a casing for containing said input/output device and said auxiliary device;
wherein said holder chamber is separate from said casing.

14. An adapter as defined in claim 1, wherein a number of signal lines between said ultrasonic diagnosis device and said diagnosis device connector is smaller than a number of signal lines between said second ultrasonic probe and said probe connector.

15. An adapter as defined in claim 14, wherein said input/output device includes a multiplexer and lines for interconnecting said multiplexer and said signal lines, and said multiplexer changes over line connection between said signal lines.

16. An ultrasonic diagnosis system, comprising:
an ultrasonic diagnosis device (30) for diagnosis according to a signal from a first ultrasonic probe (60) for extracorporeal use;
a second ultrasonic probe (10, 82) for in-vivo diagnosis to detect an object; and
an adapter for inputting and/or outputting between said ultrasonic diagnosis device and said second ultrasonic probe;
said adapter including:
a holder chamber (71, 186) for holding said ultrasonic diagnosis device;
a diagnosis device connector (74) for connection with said ultrasonic diagnosis device;
a probe connector (72) for connection with said second ultrasonic probe;
an input/output device (120) for transmission and/or reception between said probe connector and said diagnosis device connector;
an auxiliary device (122) for performance auxiliary to said ultrasonic diagnosis device.

17. An ultrasonic diagnosis system as defined in claim 16, wherein said diagnosis device connector is connectable with a connection terminal of said ultrasonic diagnosis device for said first ultrasonic probe.
